# EUROPÄISCHE PATENTSCHRIFT

(11) **EP 2 374 495 B1**
(45) Veröffentlichungstag und Bekanntmachung des Hinweises auf die Patenterteilung: **30.09.2015**
(21) Anmeldenummer: 10159449.7
(22) Anmeldetag: 09.04.2010
(51) Int. Cl.: A61M 16/12, A61M 16/10, B01F 3/02, B01F 15/02

(54) **Beatmungsvorrichtung**
Ventilation device
Dispositif respiratoire

(43) Veröffentlichungstag der Anmeldung: 12.10.2011
(73) Patentinhaber: Dräger Medical GmbH, 23558 Lübeck (DE)
(72) Erfinder: Heesch, Ralf, 23568 Lübeck (DE); Brandt, Andreas, 23554 Lübeck (DE)
(74) Vertreter: UEXKÜLL & STOLBERG

(56) Entgegenhaltungen:
- WO-A1-98/29154
- WO-A2-2007/064986
- US-A- 4 023 587
- US-A- 5 383 449
- US-A- 5 915 834

## Beschreibung

Die vorliegende Erfindung betrifft eine Beatmungs- oder Anästhesiegerät gemäß dem Oberbegriff des Anspruches 1 und ein Verfahren zum Mischen von wenigstens zwei verschiedenen Gasen in einem Beatmungs- und Anästhesiegeräte gemäß dem Oberbegriff des Anspruches 7.

Aus US 5,383,449 A und WO 2007/064986 A2 sind jeweils Beatmungsgeräte mit den Merkmalen des Oberbegriffs von Patentanspruch 1 und Verfahren mit den Merkmalen des Oberbegriffs von Anspruch 7 bekannt.

Für verschiedene medizinische Anwendungen, z. B. bei Operationen, ist eine künstliche Beatmung von Patienten notwendig. Beatmungsgeräte dienen zur künstlichen Beatmung von Patienten und können zusätzlichen mit einem Anästhesiemittelreflektor und Anästhesiemitteldosierer als Anästhesiegeräte auch zur Narkose eingesetzt werden. Bei einigen Beatmungsgeräten kann wenigstens teilweise das von dem Patienten ausgeatmete Exspirationsgas wieder als Inspirationsgas wiederverwendet werden, d. h. es handelt sich um ein Rückatemsystem mit einem Atemluftkreissystem. In dem Beatmungsgerät mit dem Atemluftkreissystem ist eine Gasfördereinrichtung vorhanden, welche die Atemluft während der Inspiration zum Patienten leitet. Während und nach der Ausatmung ist die Gasfördereinrichtung entweder abgeschaltet oder wird nur mit einem sehr geringen Förderstrom betrieben.

Insbesondere bei Anästhesiegeräten ist es erforderlich, dem Inspirationsgas ein Mischgas zuzuführen. Das Mischgas besteht dabei beispielsweise aus Sauerstoff und Lachgas. Dabei werden die zu mischenden Gase, nämlich Sauerstoff und Lachgas, getrennt durch Absperrventile einem Mischgastank zugeführt. Das Einleiten der zu mischenden Gase, beispielsweise Sauerstoff oder Lachgas, erfolgt dabei diskontinuierlich dahingehend, dass abwechselnd nur ein Absperrventil geöffnet ist. Es wird somit dem Mischgastank entweder beispielsweise nur Sauerstoff oder nur Lachgas zugeführt. Das Ausleiten des Mischgases aus dem Mischgastank erfolgt dabei kontinuierlich in ein Atemluftleitungssystem, insbesondere in eine Inspirationsgasleitung. Wird ein zu mischendes Gas, beispielsweise Sauerstoff, in den Mischgastank eingeleitet, erhöht sich die Konzentration an Sauerstoff in dem Mischgastank, weil nur Sauerstoff in den Mischgastank eingeleitet wird. Dies gilt auch in analoger Weise, sofern in den Mischgastank beispielsweise nur Lachgas eingeleitet wird. Das Ausleiten des Mischgases aus dem Mischgastank erfolgt dabei kontinuierlich. Das aus dem Mischgastank ausgeleitete Mischgas wird einem Atemluftleitungssystem eines Beatmungs- oder Anästhesiegerätes zugeführt. Auf Grund der diskontinuierlichen Dosierung kommt es in dem Mischgastank und somit auch dem Mischgas, welches dem Atemluftleitungssystem zugeführt wird, zu Konzentrationsschwankungen. Diese Konzentrationsschwankungen sind jedoch nicht gewünscht, weil das dem Patienten zugeführte Inspirationsgas eine möglichst gleichmäßige Konzentration aufweisen soll.

Innerhalb des Mischgastanks können auch lokale Konzentrationsschwankungen auftreten, so dass dadurch aufgrund einer nicht optimalen Vermischung des Mischgases in dem Mischgastank ebenfalls zusätzliche Konzentrationsschwankungen des dem Atemluftleitungssystem zugeführten Mischgases auftreten können.

Die Aufgabe der vorliegenden Erfindung besteht deshalb darin, ein Beatmungs- oder Anästhesiegerät und ein Verfahren zum Mischen von wenigstens zwei verschiedenen Gasen in einem Beatmungs- und Anästhesiegeräte zur Verfügung zu stellen, bei dem Konzentrationsschwankungen eines in ein Atemluftleitungssystem eingeleiteten Mischgases einfach und zuverlässig mit einem geringen technischen Aufwand ausgeglichen werden können.

Diese Aufgabe wird gelöst mit einem Beatmungs- oder Anästhesiegerät zur künstlichen Beatmung eines Patienten, umfassend eine Gasfördereinrichtung, wenigstens eine Gasleitung zur Ausbildung eines Atemluftleitungssystems, insbesondere eines Atemluftkreissystems, wenigstens eine Gasmischeinrichtung mit einem Mischgastank und wenigstens zwei Einlassöffnungen mit je einem Absperrorgan zur getrennten Zuführung von wenigstens zwei verschiedenen zu mischenden Gasen, insbesondere Sauerstoff, Lachgas, Luft, Kohlendioxid oder Xenon, in den Mischgastank und zur anschließenden Zuführung des Mischgases aus dem Mischgastank in das Atemluftleitungssystem, wobei der Mischgastank mit wenigstens zwei separaten Auslassöffnungen versehen ist zum Ausleiten des Mischgases aus dem Mischgastank und zur anschließenden Zuführung in das Atemluftleitungssystem. Aufgrund des Ausleitens des Mischgases aus dem Mischgastank aus wenigstens zwei separaten Auslassöffnungen können lokale Konzentrationsschwankungen innerhalb des Mischgastankes ausgeglichen werden.

An den wenigstens zwei Auslassöffnungen ist je eine Mischgasleitung angeordnet, so dass das aus den wenigstens zwei Auslassöffnungen ausgeleitete Mischgas vor dem Einleiten in das Atemluftleitungssystem durch wenigstens zwei Mischgasleitungen strömt.

Die wenigstens zwei Mischgasleitungen münden in eine Mischgassammelleitung und die Mischgassammelleitung mündet in das Atemluftleitungssystem. In der Mischgassammelleitung erfolgt eine zusätzliche Vermischung der Gase des Mischgases.

Die wenigstens zwei Mischgasleitungen weisen ein unterschiedliches Volumen für eine pneumatische Zeitverzögerung zwischen den wenigstens zwei Auslassöffnungen und dem Atemluftleitungssystem und/oder der Mischgassammelleitung auf. Innerhalb des Mischgases in dem Mischgastank treten Konzentrationsschwankungen der dem Mischgastank zugeführten zu mischenden Gase auf. Dabei kann dem Mischgastank abwechselnd jeweils nur ein zu mischendes Gas zugeführt werden, so dass es zu Konzentrationsschwankungen in dem Mischgas im Mischgastank kommt. Durch eine pneumatische zeitliche Verzögerung des aus den wenigstens zwei Auslassöffnungen entnommenen Mischgases können die Konzentrationsschwankungen der unterschiedlichen dem Mischgastank zugeführten zu mischenden Gase im Wesentlichen ausgeglichen werden. Liegt beispielsweise ein erstes zu mischendes Gas zu einem ersten Zeitpunkt in dem Mischgastank in einer maximalen Konzentration vor und ein zweites zu mischendes Gas in dem Mischgastank zu einem zweiten Zeitpunkt in einer maximalen Konzentration vor, entspricht die pneumatische Zeitverzögerung im Wesentlichen der Zeitdifferenz zwischen dem ersten und zweiten Zeitpunkt. Während des zweiten Zeitpunktes liegt das erste zu mischende Gas in einer minimalen Konzentration in dem Mischgastank vor. Dabei wird beispielsweise das Gas, welches während dem ersten Zeitpunkt dem Mischgastank entnommen worden ist, mit dem Mischgas vermischt, welches während des zweiten Zeitpunktes dem Mischgastank entnommen worden ist. Dadurch wird Mischgas mit einer maximalen Konzentration von dem zweiten zu mischenden Gas mit Mischgas vermischt, welches eine maximale Konzentration von dem ersten zu mischenden Gas aufweist. Aufgrund der kontinuierlich an- und absteigenden Konzentrationsschwankungen des ersten und zweiten zu mischenden Gases in dem Mischgastank können dadurch die auftretenden Konzentrationsschwankungen in dem Mischgastank im Wesentlichen ausgeglichen werden bezüglich des dem Atemluftleitungssystem zugeführten Mischgases.

In einer ergänzenden Ausgestaltung weisen die wenigstens zwei separaten Auslassöffnungen in dem Mischgastank einen Abstand von wenigstens 1 cm, 2 cm, 5 cm, 7 cm oder 10 cm auf.

Vorzugsweise ist das Volumen wenigstens einer Mischgasleitung wenigstens um das 1,1-, 1,5-, 2-, 3- oder 5-Fache größer als das Volumen einer anderen Mischgasleitung.

In einer Variante sind die durch die wenigstens zwei Einlassöffnungen mit je einem Absperrorgan in den Mischgastank eingeleiteten zu mischenden Gasen in den Mischgastank diskontinuierlich einleitbar.

Zweckmäßig sind die Absperrorgane dahingehend gesteuert, dass abwechselnd nur je ein Absperrorgan geöffnet ist.

In einer weiteren Ausführungsform entspricht die Differenz des Volumens von wenigstens zwei Mischgasleitungen im Wesentlichen einer Abweichung von weniger als 70 %, 50 %, 30 % oder 20 % dem Volumen des gemischten Gases, das während einer Öffnungszeit eines Absperrorganes durch die Auslassöffnung strömt, welche an der Mischgasleitung mit dem größeren Volumen angeordnet ist.

Insbesondere ist wenigstens eine Mischgasleitung wenigstens teilweise, insbesondere als Puffertank, innerhalb des Mischgastankes angeordnet. Die wenigstens eine Mischgasleitung mit dem größeren Volumen kann damit kompakt aufgebaut werden, weil diese dadurch in den Mischgastank als Puffertank integriert ist und damit insgesamt die Gasmischeinrichtung einen kompakten Aufbau aufweist.

Erfindungsgemäßes Verfahren zum Mischen von wenigstens zwei verschiedenen Gasen in eines in dieser Schutzrechtsanmeldung beschriebenen Beatmungs- oder Anästhesiegerätes, mit den Schritten: Einleiten von wenigstens zwei zu mischenden Gasen in einen Mischgastank, Vermischen der wenigstens zwei Gase in dem Mischgastank, Ausleiten des Mischgases aus dem Mischgastank, Einleiten des Mischgases in ein Atemluftleitungssystem, wobei das Mischgas aus dem Mischgastank getrennt an wenigstens zwei verschiedenen Auslassöffnungen ausgeleitet wird.

Dabei fließt Gas aus den zwei Auslassleitungen jeweils durch eine von an die wenigstens zwei Auslassöffnungen anschließenden Mischgasleitungen. Darauf folgt ein Zusammenführen der Strömungen durch die Mischgasleitungen in eine Mischgassammelleitung und Weiterleiten durch die Mischgassammelleitung in das Atemluftleitungssystem, wobei die wenigstens zwei Mischgasleitungen ein unterschiedliches Volumen aufweisen für eine pneumatische Zeitverzögerung zwischen den wenigstens zwei Auslassöffnungen und dem Atemluftleitungssystem und/oder der Mischgassammelleitung.

In einer weiteren Ausgestaltung wird das aus einer Auslassöffnung ausgeleitete Mischgas kontinuierlich mit einer Zeitdifferenz dem Atemluftleitungssystem zugeführt bezüglich dem aus einer anderen Auslassöffnung ausgeleiteten und dem Atemluftleitungssystem zugeführten Mischgas.

Es zeigt:

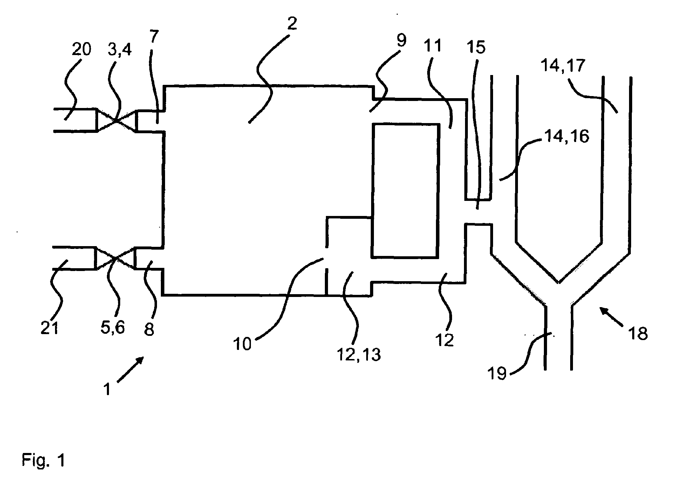

- Fig. 1: einen vereinfachte Darstellung einer Gasmischeinrichtung.

In Fig. 1 ist eine Gasmischeinrichtung 1 eines nicht dargestellten Anästhesiegerätes abgebildet.

Das Anästhesiegerät weist ein Atemluftkreissystem auf, so dass von dem Patienten ausgeatmetes Exspirationsgas wenigstens teilweise als Inspirationsgas wiederverwendet werden kann. Das Anästhesiegerät dient somit zur künstlichen Beatmung und zur Narkose eines nicht dargestellten Patienten.

Das dem zu beatmenden Patienten zugeführte Inspirationsgas wird dem Patienten durch eine Inspirationsgasleitung 16 als Bestandteil eines Atemluftleitungssystems 14, insbesondere hier als Atemluftkreissystem ausgeführt, zugeführt. Nach dem Durchströmen der Inspirationsgasleitung 16 und eines Y-Stückes 18 wird das Inspirationsgas durch ein Mundstück 19 dem Patienten zugeführt. Die von dem Patienten ausgeatmete Luft als Exspirationsgas wird durch eine Exspirationsgasleitung 17 abgeleitet. Dabei sind an der Inspirationsgas- als auch an der Exspirationsgasleitung 16, 17 jeweils nicht dargestellte Rückschlagventile angeordnet.

In die Inspirationsgasleitung 16 mündet eine Mischgassammelleitung 15. Durch die Mischgasleitung 15 wird Mischgas aus verschiedenen Gasen wie beispielsweise Sauerstoff und Lachgas dem Inspirationsgas für den zu beatmenden Patienten zugeführt. Die Gasmischeinrichtung 1, welche das Mischgas zur Verfügung stellt, welches durch die Mischgassammelleitung 15 in die Inspirationsgasleitung 16 eingeführt wird, weist einen Mischgastank 2 mit einer ersten Einlassöffnung 7 und einer zweiten Einlassöffnung 8 auf. An der ersten Einlassöffnung 7 ist ein erstes Absperrorgan 3, beispielsweise ein Absperrventil 4 oder ein Absperrschieber angeordnet, und an der zweiten Einlassöffnung 8 ist ein zweites Absperrorgan 5, beispielsweise ein zweites Absperrventil 6 oder ein zweiter Absperrschieber. Durch eine erste Zuführleitung 20 wird ein erstes zu mischendes Gas, z. B. Sauerstoff, in den Mischgastank 2 eingeleitet und durch eine zweite Zuführleitung 21 ein zweites zu mischendes Gas, z. B. Lachgas, in den Mischgastank 2 eingeleitet. Dabei wird das erste Absperrorgan 3 und das zweite Absperrorgan 5 abwechselnd geöffnet, so dass durch die erste Einlassöffnung 7 zu einem Zeitabschnitt nur Sauerstoff in den Mischgastank 2 einströmt und zu einem zweiten Zeitabschnitt nur Lachgas durch die zweite Einlassöffnung 8 in den Mischgastank 2 eingeleitet wird. Die beiden Absperrorgane 3, 5 sind somit nicht gleichzeitig geöffnet.

Der Mischgastank 2 weist eine erste Auslassöffnung 9 und eine zweite Auslassöffnung 10 auf. Durch die erste und zweite Auslassöffnung 9, 10 wird das in dem Mischgastank gemischte Gas aus dem Mischgastank 2 ausgeleitet. Dabei weisen die beiden Auslassöffnungen 9, 10 einen größeren Abstand, beispielsweise im Bereich von 1 cm bis 10 cm, auf. Das aus der ersten Auslassöffnung 9 ausgeleitete Mischgas wird durch eine erste Mischgasleitung 11 der Mischgassammelleitung 15 zugeführt und das aus der zweiten Auslassöffnung 10 ausgeleitete Mischgas wird durch eine zweite Mischgasleitung 12 ebenfalls zu der Mischgassammelleitung 15 geleitet. Die zweite Mischgasleitung 12 ist dabei auch teilweise in den Mischgastank 2 integriert und weist ein wesentlich größeres Volumen auf als die erste Mischgasleitung 11. Dabei ist die zweite Mischgasleitung 12 in dem Mischgastank 2 als Puffertank 13 ausgebildet, an dem auch die zweite Auslassöffnung 10 vorhanden ist. Aufgrund des getrennten und separaten Ausleitens des Mischgases aus dem Mischgastank durch zwei verschiedene Auslassöffnungen 9, 10 können lokale Konzentrationsschwankungen an Sauerstoff und Lachgas innerhalb des Mischgastankes 2 in vorteilhafter Weise im Wesentlichen ausgeglichen werden.

Aufgrund der diskontinuierlichen und abwechselnden Einleitung der beiden zu mischenden Gase in den Mischgastank 1 treten Konzentrationsschwankungen der beiden Gase Sauerstoff und Lachgas in dem Mischgas auf. Wird beispielsweise nur Sauerstoff durch das erste Absperrorgan 3 und die erste Einlassöffnung 7 in den Mischgastank 2 eingeleitet, steigt die Konzentration an Sauerstoff in dem Mischgastank 2 an und dadurch sinkt auch die Konzentration an Lachgas in dem Mischgastank 2. Dabei wird das Mischgas aus den beiden Auslassöffnungen 9, 10 kontinuierlich aus dem Mischgastank 2 ausgeleitet und kontinuierlich in die Inspirationsgasleitung 16 eingeleitet. Das Volumen der zweiten Mischgasleitung 12 mit dem Puffertank 13 ist dabei wesentlich größer als das Volumen der ersten Mischgasleitung 11. Dies führt zu einer pneumatischen Zeitverzögerung des Mischgases, welches durch die erste und zweite Mischgasleitung 11, 12 in die Mischgassammelleitung 15 einströmt. Die Differenz des Volumens zwischen der ersten und zweiten Mischgasleitung 11,12 ist dabei dahingehend ausgelegt, dass das durch die erste Mischgasleitung 11 zu der Mischgassammelleitung 15 geleitete Mischgas eine maximale Konzentration an Sauerstoff aufweist und zum gleichen Zeitpunkt das durch die zweite Mischgasleitung 12 in die Mischgassammelleitung 15 eingeleitete Mischgas eine maximale Konzentration an Lachgas aufweist. Aufgrund des größeren Volumens der zweiten Mischgasleitung 12 strömt somit das Mischgas, welches durch die zweite Mischgasleitung 12 aus dem Mischgastank 2 ausgeleitet wird und in die Mischgassammelleitung 15 eingeleitet wird, mit einer zeitlichen Verzögerung in die Mischgassammelleitung 15 bezüglich des Mischgases, welches durch die erste Mischgasleitung 11 in die Mischgassammelleitung 15 eingeleitet wird. Bei einer maximalen Konzentration von Sauerstoff in dem Mischgastank 2 liegt eine minimale Konzentration von Lachgas in dem Mischgastank 2 vor und umgekehrt. Während der ausschließlichen Zuführung von nur Sauerstoff in den Mischgastank 2 steigt kontinuierlich die Konzentration an Sauerstoff in dem Mischgastank 2 an und in analoger Weise sinkt die Konzentration an Lachgas in dem Mischgastank 2. In analoger Weise steigt beim ausschließlichen Einleiten von Lachgas in den Mischgastank 2 kontinuierlich die Konzentration an Lachgas in dem Mischgastank 2 an und die Konzentration an Sauerstoff in dem Mischgastank 2 sinkt ab. Sinkt beispielsweise in dem Mischgastank 2 die Konzentration an Sauerstoff und steigt die Konzentration an Lachgas, führt dies aufgrund der pneumatischen Verzögerung zu Beginn der Mischgassammelleitung 15 dazu, dass bei einer ansteigenden Konzentration an Sauerstoff des Mischgases am Ende der ersten Mischgasleitung 11 an der Mischgassammelleitung 15 gleichzeitig die Konzentration des Mischgases in der zweiten Mischgasleitung 12, d. h. dem Ende der zweiten Mischgasleitung 12 an der Mischgassammelleitung 15, die Konzentration an Lachgas zunimmt. Dies gilt auch umgekehrt. Damit können kontinuierlich die Konzentrationsschwankungen des Mischgases in dem Mischgastank 2 aufgrund der abwechselnden und diskontinuierlichen Einleitung der beiden zu mischenden Gase in dem Mischgastank 2 im Wesentlichen auf einfache Art und Weise ausgeglichen werden.

Insgesamt betrachtet sind mit dem erfindungsgemäßen Beatmungs- oder Anästhesiegerät wesentliche Vorteile verbunden. Die getrennte Ausleitung des Mischgases aus wenigstens zwei separaten Auslassöffnungen 9, 10 ermöglicht es, lokale Konzentrationsschwankungen innerhalb des Mischgastankes 2 aufgrund einer nicht homogenen Durchmischung des Mischgases im Wesentlichen auszugleichen. Die zeitlichen Schwankungen der Konzentration der zu mischenden Gase in dem Mischgastank 2 aufgrund der diskontinuierlichen Einleitung dieser in den Mischgastank 2 können durch die pneumatische Zeitverzögerung in den beiden Mischgasleitungen 11, 12 im Wesentlichen ausgeglichen werden. Dies ermöglicht eine im Wesentlichen konstante Konzentration des der Inspirationsgasleitung 16 zugeführten Mischgases.

### BEZUGSZEICHENLISTE

- 1: Gasmischeinrichtung
- 2: Mischgastank
- 3: Erstes Absperrorgan
- 4: Erstes Absperrventil
- 5: Zweites Absperrorgan
- 6: Zweites Absperrventil
- 7: Erste Einlassöffnung
- 8: Zweite Einlassöffnung
- 9: Erste Auslassöffnung
- 10: Zweite Auslassöffnung
- 11: Erste Mischgasleitung
- 12: Zweite Mischgasleitung
- 13: Puffertank
- 14: Atemluftleitungssystem
- 15: Mischgassammelleitung
- 16: Inspirationsgasleitung
- 17: Exspirationsgasleitung
- 18: Y-Stück
- 19: Mundstück
- 20: Erste Zuführleitung für das erste zu mischende Gas
- 21: Zweite Zuführleitung für das zweite zu mischende Gas

## Patentansprüche

1. Beatmungs- oder Anästhesiegerät zur künstlichen Beatmung eines Patienten, umfassend
eine Gasfördereinrichtung,
wenigstens eine Gasleitung zur Ausbildung eines Atemluftleitungssystems (14), insbesondere eines Atemluftkreissystems,
wenigstens eine Gasmischeinrichtung (1) mit einem Mischgastank (2) und wenigstens zwei Einlassöffnungen (7, 8) mit je einem Absperrorgan (3, 5) zur getrennten Zuführung von wenigstens zwei verschiedenen zu mischenden Gasen in den Mischgastank (2) und zur anschließenden Zuführung des Mischgases in das Atemluftleitungssystem (14), wobei der Mischgastank (2) mit wenigstens zwei separaten Auslassöffnungen (9, 10) versehen ist zum Ausleiten des Mischgases aus dem Mischgastank (2) und zur anschließenden Zuführung in das Atemluftleitungssystem (14), wobei an den wenigstens zwei Auslassöffnungen (9, 10) je eine Mischgasleitung (11, 12) angeordnet ist, so dass das aus den wenigstens zwei Auslassöffnungen (9, 10) ausgeleitete Mischgas vor dem Einleiten in das Atemluftleitungssystem (14) durch die wenigstens zwei Mischgasleitungen (11, 12) strömt,
**dadurch gekennzeichnet, dass**
die wenigstens zwei Mischgasleitungen (11, 12) in eine Mischgassammelleitung (15) münden und die Mischgassammelleitung (15) in das Atemluftleitungssystem (14) mündet und
dass die wenigstens zwei Mischgasleitungen (11, 12) ein unterschiedliches Volumen aufweisen für eine pneumatische Zeitverzögerung zwischen den wenigstens zwei Auslassöffnungen (9, 10) und dem Atemluftleitungssystem (14) und/oder der Mischgassammelleitung (15).

2. Beatmungs- oder Anästhesiegerät nach Anspruch 1, **dadurch gekennzeichnet, dass** das Volumen wenigstens einer Mischgasleitung (11, 12) größer ist als das Volumen einer anderen Mischgasleitung (11, 12), vorzugsweise um das wenigstens 1,1-, 1,5-, 2-, 3- oder 5-fache.

3. Beatmungs- oder Anästhesiegerät nach einem der vorhergehenden Ansprüche, **dadurch gekennzeichnet, dass** die durch die wenigstens zwei Einlassöffnungen (7, 8) mit je einem Absperrorgan (3, 5) in den Mischgastank (2) eingeleiteten zu mischenden Gase in den Mischgastank (2) diskontinuierlich einleitbar sind.

4. Beatmungs- oder Anästhesiegerät nach einem oder mehreren der vorhergehenden Ansprüche, **dadurch gekennzeichnet, dass** die Absperrorgane (3, 5) dahingehend gesteuert sind, dass abwechselnd nur je ein Absperrorgan (3, 5) geöffnet ist.

5. Beatmungs- oder Anästhesiegerät nach einem oder mehreren der vorhergehenden Ansprüche, **dadurch gekennzeichnet, dass** die Differenz des Volumens von wenigstens zwei Mischgasleitungen (11, 12) im Wesentlichen, zum Beispiel mit einer Abweichung von weniger als 70%, 50%, 30% oder 20%, dem Volumen des gemischten Gases entspricht, das während einer Öffnungszeit eines Absperrorgans (3, 5) durch die Auslassöffnung (9, 10) strömt, welche an der Mischgasleitung (11, 12) mit dem grö0eren Volumen angeordnet ist.

6. Beatmungs- oder Anästhesiegerät nach einem oder mehreren der Ansprüche 2 bis 8, **dadurch gekennzeichnet, dass** wenigstens eine Mischgasleitung (11, 12) wenigstens teilweise, insbesondere als Puffertank (13), innerhalb des Mischgastankes (2) angeordnet ist.

7. Verfahren zum Mischen von wenigstens zwei verschiedenen Gasen in einem Beatmungs- oder Anästhesiegerät nach einem oder mehreren der vorhergehenden Ansprüche, mit den Schritten:
Einleiten von wenigstens zwei zu mischenden Gasen in einen Mischgastank (2),
Vermischen der wenigstens zwei Gase in dem Mischgastank (2),
Ausleiten des Mischgases aus dem Mischgastank (2) getrennt an wenigstens zwei verschiedenen Auslassöffnungen (9, 10), jeweils durch eine von an die wenigstens zwei Auslassöffnungen anschließenden Mischgasleitungen (11, 12),
Einleiten des Mischgases in ein Atemluftleitungssystem (14),
**gekennzeichnet durch** die Schritte
Zusammenführen der Strömungen **durch** die Mischgasleitungen (11, 12) in eine Mischgassammelleitung (15) und Weiterleiten **durch** die Mischgassammelleitung (15) in das Atemluftleitungssystem (14), wobei die wenigstens zwei Mischgasleitungen (11, 12) ein unterschiedliches Volumen aufweisen für eine pneumatische Zeitverzögerung zwischen den wenigstens zwei Auslassöffnungen (9, 10) und dem Atem-luftleitungssystem (14) und/oder der Mischgassammelleitung (15).

8. Verfahren nach Anspruch 7, **dadurch gekennzeichnet, dass** das aus einer Auslassöffnung (9, 10) ausgeleitete Mischgas kontinuierlich mit einer Zeitdifferenz dem Atemluftleitungssystem (14) zugeführt wird bezüglich dem aus einer anderen Auslassöffnung (9, 10) ausgeleiteten und dem Atemluftleitungssystem (14) zugeführten Mischgas.

## Claims

1. Respirator or anesthesia apparatus for the artificial respiration of a patient, the respirator or anesthesia apparatus comprising:
a gas delivery means;
at least one gas line for forming a breathing air line system (14), in particular a breathing air circulation system;
at least one gas mixing device (1) with a mixed gas tank (2) and at least two inlet openings (7, 8), each of the inlet openings having a shut-off member (3, 5) for separately feeding at least two different gases to be mixed into the mixed gas tank (2) and for subsequently feeding the mixed gas into the breathing air line system (14), the mixed gas tank (2) having at least two separate outlet openings (9, 10) for releasing the mixed gas from the mixed gas tank and for subsequently feeding mixed gas into the breathing air line system (14), wherein at each of the at least two outlet openings (9, 10) a mixed gas line (11, 12) is arranged so that mixed gas released from the at least two outlet openings (9, 10) flows through the at least two mixed gas lines (11, 12) before being introduced into the breathing air line system (14),
**characterized in that**
the at least two mixed gas lines (11, 12) open into a mixed gas collection line (15) and the mixed gas collection line (15) opens into the breathing air line system (14), and that the at least two mixed gas lines (11, 12) have different volumes for a pneumatic time delay between the at least two outlet openings (9, 10) and the breathing air line system (14) and/or the mixed gas collection line (15).

2. Respirator or anesthesia apparatus according to claim 1, **characterized in that** the volume of at least one of the mixed gas lines (11, 12) is larger than the volume of another of the mixed gas lines (11, 12), preferably by at least 1,1, 1,5, 2, 3 or 5 times.

3. Respirator or anesthesia apparatus according to any of the preceding claims, **characterized in that** the gases to be mixed, which are introduced into the mixed gas tank (2) through the at least two inlet openings (7, 8), each having one of the shut-off members (3, 5), can be introduced into the mixed gas tank (2) intermittently.

4. Respirator or anesthesia apparatus according to one or more of the preceding claims, **characterized in that** the shut-off members (3, 5) are controlled such that only one shut-off member (3, 5) is alternatingly opened.

5. Respirator or anesthesia apparatus according to one or more of the preceding claims, **characterized in that** the difference of the volumes of at least two mixed gas lines (11, 12) essentially corresponds, for example with a deviation of less than 70%, 50%, 30% or 20%, to the volume of the mixed gas which flows during the opening time of a shut-off member (3, 5) through the outlet opening at which the mixed gas line (11, 12) with the larger volume is arranged.

6. Respirator or anesthesia apparatus according to one or more of claims 2 to 8, **characterized in that** at least one mixed gas line (11, 12) is arranged at least partly within the mixed gas tank (2), in particular as a buffer tank (13).

7. Process for mixing at least two different gases in a respirator or anesthesia apparatus according to one or more of the preceding claims, comprising the steps of:
introducing at least two gases to be mixed into a mixed gas tank (2);
mixing the at least two gases in the mixed gas tank (2);
releasing the mixed gas from the mixed gas tank (2) separately at at least two different outlet openings (9, 10), in each case through one of the at least two mixed gas lines (11, 12) connected to the at least two outlet openings;
introducing the mixed gas into the breathing air line system (14);
**characterized by** the steps of:
merging the flows through the mixed gas lines (11, 12) into a mixed gas collection line (15) and further conveying the mixed gas through the mixed gas collection line (15) into the breathing air line system (14), wherein the at least two mixed gas lines (11, 12) have different volumes for a pneumatic time delay between the at least two outlet openings (9, 10) and the breathing air line system (14) and/or the mixed gas collection line (15).

8. Process according to claim 7, **characterized in that** the mixed gas discharged from an outlet opening (9, 10) is introduced into the breathing air line system (14) with a time difference with respect to the mixed gas discharged from another outlet opening (9, 10) and conveyed to the breathing air line system (14).

## Revendications

1. Appareil respiratoire ou d'anesthésie pour la respiration artificielle d'un patient, comprenant
un dispositif de transport de gaz,
au moins une conduite de gaz pour réaliser un système de conduite d'air respiratoire (14), en particulier un système de circuit d'air respiratoire,
au moins un dispositif de mélange de gaz (1) avec un réservoir de gaz de mélange (2) et au moins deux ouvertures d'entrée (7, 8) ayant chacune un organe d'arrêt (3, 5) pour l'alimentation séparée d'au moins deux gaz différents à mélanger dans le réservoir de gaz de mélange (2) et pour l'alimentation subséquente du gaz mélangé dans le système de conduite d'air respiratoire (14), le réservoir de gaz de mélange (2) étant muni d'au moins deux ouvertures de sortie séparées (9, 10) pour la sortie du gaz mélangé hors du réservoir de gaz de mélange (2) et pour l'alimentation subséquente dans le système de conduite d'air respiratoire (14), une conduite de gaz mélangé (11, 12) étant disposée à chaque fois au niveau des au moins deux ouvertures de sortie (9, 10), de sorte que le gaz mélangé guidé hors des au moins deux ouvertures de sortie (9, 10) s'écoule avant son introduction dans le système de conduite d'air respiratoire (14) à travers les au moins deux conduites de gaz mélangé (11, 12),
**caractérisé en ce que**
les au moins deux conduites de gaz mélangé (11, 12) débouchent dans une conduite collectrice de gaz mélangé (15) et la conduite collectrice de gaz mélangé (15) débouche dans le système de conduite d'air respiratoire (14) et **en ce que** les au moins deux conduites de gaz mélangé (11, 12) présentent un volume différent pour un retard temporel pneumatique entre les au moins deux ouvertures de sortie (9, 10) et le système de conduite d'air respiratoire (14) et/ou la conduite collectrice de gaz mélangé (15).

2. Appareil respiratoire ou d'anesthésie selon la revendication 1, **caractérisé en ce que** le volume d'au moins une conduite de gaz mélangé (11, 12) est supérieur au volume d'une autre conduite de gaz mélangé (11, 12), de préférence d'un facteur d'au moins 1,1, 1,5, 2, 3 ou 5.

3. Appareil respiratoire ou d'anesthésie selon l'une quelconque des revendications précédentes, **caractérisé en ce que** les gaz à mélanger introduits à travers les au moins deux ouvertures d'entrée (7, 8) munies chacune d'un organe d'arrêt (3, 5) dans le réservoir de gaz de mélange (2) peuvent être introduits dans le réservoir de gaz de mélange (2) de manière discontinue.

4. Appareil respiratoire ou d'anesthésie selon l'une quelconque ou plusieurs des revendications précédentes, **caractérisé en ce que** les organes d'arrêt (3, 5) sont commandés de manière à ce qu'un organe d'arrêt respectif (3, 5) soit seulement ouvert en alternance.

5. Appareil respiratoire ou d'anesthésie selon l'une quelconque ou plusieurs des revendications précédentes, **caractérisé en ce que** la différence du volume d'au moins deux conduites de gaz mélangé (11, 12) correspond essentiellement, par exemple avec un écart inférieur à 70 %, 50 %, 30 % ou 20 %, au volume du gaz mélangé, qui s'écoule pendant un temps d'ouverture d'un organe d'arrêt (3, 5) à travers l'ouverture de sortie (9, 10) qui est disposée au niveau de la conduite de gaz mélangé (11, 12) ayant le plus grand volume.

6. Appareil respiratoire ou d'anesthésie selon l'une quelconque ou plusieurs des revendications 2 à 8, **caractérisé en ce qu'**au moins une conduite de gaz mélangé (11, 12) est disposée au moins en partie, notamment sous la forme d'un réservoir tampon (13), à l'intérieur du réservoir de gaz de mélange (2).

7. Procédé pour mélanger au moins deux gaz différents dans un appareil respiratoire ou d'anesthésie, en particulier un appareil respiratoire ou d'anesthésie selon l'une quelconque ou plusieurs des revendications précédentes, comprenant les étapes suivantes :
introduction d'au moins deux gaz à mélanger dans un réservoir de gaz de mélange (2),
mélange des au moins deux gaz dans le réservoir de gaz de mélange (2),
guidage du gaz mélangé hors du réservoir de gaz de mélange (2) de manière séparée au niveau d'au moins deux ouvertures de sortie différentes (9, 10), à chaque fois par l'une de conduites de gaz mélangé (11, 12) se raccordant aux au moins deux ouvertures de sortie,
introduction du gaz mélangé dans un système de conduite d'air respiratoire (14),
**caractérisé par** les étapes suivantes
réunion des écoulements à travers les conduites de gaz mélangé (11, 12) dans une conduite collectrice de gaz mélangé (15) et guidage subséquent à travers la conduite collectrice de gaz mélangé (15) dans le système de conduite d'air respiratoire (14), les au moins deux conduites de gaz mélangé (11, 12) présentant un volume différent pour un retard temporel pneumatique entre les au moins deux ouvertures de sortie (9, 10) et le système de conduite d'air respiratoire (14) et/ou la conduite collectrice de gaz mélangé (15).

8. Procédé selon la revendication 7, **caractérisé en ce que** le gaz mélangé guidé hors d'une conduite de sortie (9, 10) est acheminé de manière continue avec une différence temporelle au système de conduite d'air respiratoire (14) par rapport au gaz mélangé guidé hors d'une autre ouverture de sortie (9, 10) et acheminé au système de conduite d'air respiratoire (14).
